# EUROPEAN PATENT APPLICATION

(11) **EP 3 144 832 A1**
(43) Date of publication of application: **22.03.2017**
(21) Application number: 16160824.5
(22) Date of filing: 17.03.2016
(51) Int. Cl.: G06F 19/00

(54) **MEDICAL-DOCUMENT MANAGEMENT APPARATUS, MEDICAL-DOCUMENT MANAGEMENT SYSTEM, AND PROGRAM**

(30) Priority: 16.09.2015 JP 2015182742
(71) Applicant: Fuji Xerox Co., Ltd., Minato-ku, Tokyo (JP)
(72) Inventor: KOGURE, Yosuke, Yokohama-shi, Kanagawa (JP)
(74) Representative: Ward, James Norman

(57) **Abstract**

A medical-document management apparatus includes a recording unit and a display. The recording unit records a first medical document and link information in a database. The link information describes a link to a second medical document associated with the first medical document. The display uses the link information to give emphasis indication to the second medical document as a medical document associated with the first medical document.

## Description

### BACKGROUND

### (i) Technical Field

The present invention relates to a medical-document management apparatus, a medical-document management system, and a program.

### (ii) Related Art

In recent community medicine, attention is being focused on systems in which various medical institutions or organizations disclose medical information in order to share patient information created in the institutions.

JP-A-2015-28680 discloses a technique for obtaining time and attributes (such as a diagnostic site, treatment information, and a document) of each piece of medical data, mapping the obtained data on the same time series (X axis) and attribute items (Y axis), and displaying the mapped data for displaying medical data provided from a plurality of hospitals on one browser screen collectively.

### SUMMARY

It is effective that time and attributes of medical data provided from a plurality of hospitals are obtained, and mapped on the same time series (X axis) and attribute items (Y axis), and displayed, but searching of medical data associated with specific medical data is not sufficiently examined. Accordingly, every time medical data relating to certain medical data is searched, searching and displaying operations are required to be performed repeatedly, and time for accessing the associated medical data is required. Furthermore, when there is a huge amount of information, it cannot be accessed to the associated medical data in some cases. In addition, in a case where when mapping data on the same time series (X axis) and attribute items (Y axis), a plurality of documents is recorded in one cell, searching of a document under the case is not sufficiently examined, and searching and displaying operations are required to be performed repeatedly for confirming document details.

An object of the invention is to provide a medical-document management apparatus, a medical-document management system, and a program which are capable of performing searching of medical data associated with specific medical data more easily than that of the related art, in a case where time and attributes of medical data provided from a plurality of hospitals are obtained, and mapped two-dimensionally on the same time series (X axis) and attribute items (Y axis), and displayed.
[1] According to an aspect of the invention, there is provided a medical-document management apparatus including a recording unit and a display. The recording unit records a first medical document and link information in a database. The link information describes a link to a second medical document associated with the first medical document. The display uses the link information to give emphasis indication to the second medical document as a medical document associated with the first medical document.
[2] In the medical-document management apparatus according to [1], the display may display the first medical document and the second medical document on a two-dimensional map with time and attribute, and give emphasis indication to the first medical document and the second medical document on the two-dimensional map.
[3] In the medical-document management apparatus according to [2], the display may further display a transfer-between-hospitals history for each patient on a two-dimensional map with time and hospital, and perform the display operation in such a manner that the two-dimensional map for the medical documents is associated with the two-dimensional map for the transfer-between-hospitals history.
[4] In the medical-document management apparatus according to any one of [1] to [3], the display may further set information about whether or not emphasis indication is to be given, by using information about where the second medical document is to be published.
[5] According to another aspect of the invention, there is provided a medical-document management system including a medical-document database that stores medical documents for a plurality of hospitals, a terminal apparatus, and a medical-document management apparatus according to [2]. The medical-document management apparatus receives a medical document which is converted into an electronic format and which is transmitted from the terminal apparatus, and obtains a date, an attribute, and link information of the medical document so as to record the obtained information in the medical-document database. The link information describes a link to another medical document. In response to a view request from the terminal apparatus, the medical-document management apparatus reads a medical document from the medical-document database so as to transmit the medical document to the terminal apparatus and display the medical document on a two-dimensional map with time and attribute, and gives emphasis indication to associated medical information by using the link information.
[6] According to another aspect of the invention, there is provided a program causing a computer to execute a process including recording a first medical document and link information in a database, the link information describing a link to a second medical document associated with the first medical document, and using the link information to give emphasis indication to the second medical document as a medical document associated with the first medical document.

According to the medical-document management apparatus of [1], the medical-document management system of [5], and the program of [6], searching of the medical data associated with the specific medical data can be easily performed in comparison with a case where time and attributes of the medical data provided from a plurality of hospitals are obtained and displayed on the two-dimensional map, simply.

According to the medical-document management apparatus of [2], the associated medical document can be visually recognized in a list.

According to the medical-document management apparatus of [3], a transfer-between-hospitals history of a patient and the associated medical documents can be visually recognized in a list.

According to the medical-document management apparatus of [4], the emphasis indication can be dynamically changed depending on the specification of the instructions to which the document is to be published.

### BRIEF DESCRIPTION OF DRAWINGS

Exemplary embodiments of the present invention will be described in detail based on the following figures, wherein:
Fig. 1 is a diagram illustrating a system configuration according to an exemplary embodiment;
Fig. 2 is a diagram for describing how medical documents are processed according to the exemplary embodiment;
Fig. 3 is a diagram for describing how medical documents are processed according to the exemplary embodiment;
Fig. 4 is a diagram for describing an exemplary terminal screen according to the exemplary embodiment;
Fig. 5 is a diagram for describing an exemplary terminal screen according another exemplary embodiment;
Fig. 6A is a diagram for describing an exemplary terminal screen according to another exemplary embodiment (in which institutions to which a medical document is to be published are specified); and
Fig. 6B is a diagram for describing an exemplary terminal screen according to another exemplary embodiment (in which institutions to which a medical document is to be published are specified).

### DETAILED DESCRIPTION

Exemplary embodiments of the present invention will be described below on the basis of the drawings.

### Basic Principles

First, basic principles of the exemplary embodiments will be described.

In the exemplary embodiments, when a doctor in a certain hospital creates a medical document such as a medical record for a certain patient by referring to other medical documents which have been already created for the patient, link information in which the medical documents which have been referred to are set as a link target is attached to the created medical document. When the created medical document is recorded in a database or the like, the link information is also recorded in the database along with the medical document.

For example, a certain patient takes a medical examination in A hospital, and a doctor in A hospital creates a medical document A. In the case where the patient is transferred from A hospital to B hospital for some reason, when a doctor in B hospital creates a medical document such as a medical record, the doctor typically refers to the medical document which has been created in A hospital from which the patient has been transferred, so as to obtain necessary information about the patient.

Therefore, in the exemplary embodiments, in the case where, in creation of a medical document, a doctor refers to other medical documents, link information is created from a view record describing the view operation, and is attached to the medical document. The medical document created by the doctor in B hospital is referred to as a first medical document, and the medical document that is created by the doctor in A hospital and that is referred to when the first medical document is created is referred to as a second medical document. The first medical document and the second medical document are associated with each other by using the link information of the first medical document, and the second medical document is an associated document of the first medical document.

After that, similarly, every time a different medical document is referred to in creation of a medical document, link information is created in sequence, and is accumulated in the database or the like. As in the related art, a user may easily search for medical documents associated with a certain medical document on a two-dimensional map with time and attribute by giving emphasis indication to the associated medical documents of the certain medical document in display of medical information on the two-dimensional map.

In the exemplary embodiments, emphasis indication is given to medical documents associated with a certain medical document on the two-dimensional map. The term "emphasis indication" means that the associated medical documents are displayed so as to be easily and visually recognized relatively in comparison with the other medical documents (medical documents which are not associated with the certain medical document). Any way to show emphasis indication is employed. Examples of emphasis indication include giving emphasis indication by using the same color or in the same pattern, giving emphasis indication in increased brightness, displaying a list in a different window, and giving emphasis indication by using a pop-up menu.

Examples of emphasis indication according to the exemplary embodiments also include a practice in which any mark (for example, an arrow, a line, or a dashed line) indicating association between the first medical document and the second medical document is displayed when the first medical document is associated with the second medical document.

### Configuration

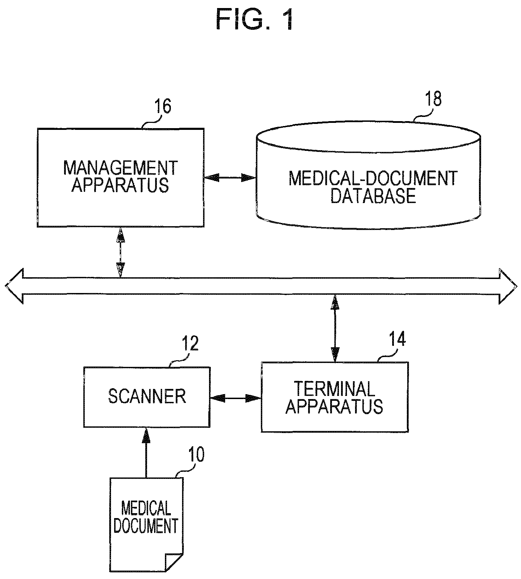

Fig. 1 is a diagram illustrating the system configuration of a medical-document management system according to an exemplary embodiment. The medical-document management system includes a scanner 12, a terminal apparatus 14, a management apparatus 16, and a medical-document database 18, and the terminal apparatus 14 and the management apparatus 16 are connected to a network. In Fig. 1, the medical-document database 18 is not connected to the network. As a matter of course, the medical-document database 18 may be connected to the network so as to be allowed to be accessed from other apparatuses.

The scanner 12 scans a medical document 10 and converts the medical document into an electronic format under the control of the terminal apparatus 14. The medical document 10 is typically a medical record created by a doctor. Other than this, any documents about medical care and diagnosis/treatment, such as a referral form, a consent form, and a notice document, may be included. Needless to say, a doctor may directly create a medical record (electronic medical record) by operating the terminal apparatus 14. The scanner 12 is desirably provided with a reading function of obtaining the creation time (date) and attributes (such as the patient name, a diagnostic site, and treatment information) of a medical document. When the date and the attributes are converted into a bar code which is printed on the medical document, the scanner 12 reads the bar code, and obtains the date and the attributes.

The terminal apparatus 14 transmits the medical document in an electronic format to the management apparatus 16. In addition, when a doctor refers to specific medical documents in creation of medical information, the terminal apparatus 14 transmits the reference information attached to the medical document as a link, to the management apparatus 16. The link may be manually attached by the doctor, or may be automatically attached by using a view record which is obtained by the terminal apparatus 14 automatically recording an operation in which the doctor views the documents. Thus, for example, when a doctor refers to a medical document B in creation of a medical document A, the following link is created:
medical document A → medical document B,
where an arrow (→) indicates a link. The number of links is not necessarily one, and may be more than one. For example, when the medical document B and a medical document C are referred to in creation of the medical document A, the following links are created:
medical document A → medical document B
medical document A → medical document C

When both of the medical document B and the medical document C are created by referring to a medical document D, the following links are created:
medical document A → medical document B → medical document D
medical document A → medical document C → medical document D
In this case, medical documents associated with the medical document A are the medical document B, the medical document C, and the medical document D.

The terminal apparatus 14 transmits a view request along with an ID for specifying the terminal apparatus 14, to the management apparatus 16 in order to view medical documents recorded in the medical-document database 18.

The management apparatus 16 records a medical document in an electronic format, along with its time (date) and attribute information, and along with its link information if any, in the medical-document database 18. In addition, in response to a view request from the terminal apparatus 14, the management apparatus 16 accesses the medical-document database 18 to obtain medical documents, and edits the medical documents before returning the medical documents to the terminal apparatus 14. In the medical-document database 18, medical documents created in multiple hospitals are recorded. The management apparatus 16 obtains the dates and attributes from medical documents, maps the medical documents on the same time series (X axis) and attribute items (Y axis), and displays the mapped data. At that time, the management apparatus 16 determines whether or not links are present among the medical documents. If links are present, the management apparatus 16 gives emphasis indication to medical documents associated with each other, in accordance with the links. In addition, the management apparatus 16 obtains a transfer-between-hospitals history for each patient, which describes transfers in which the patient is transferred from one hospital to another, from the medical documents for the patient, and edits the transfer-between-hospitals history in a graphic format before returning the transfer-between-hospitals history to the terminal apparatus 14.

The management apparatus 16 includes a medical-document receiving/transmitting unit, a medical-document recording unit, and a medical-document editing/displaying unit as functional blocks. Specifically, the management apparatus 16 is constituted by a computer (server computer), and includes a central processing unit (CPU), a program memory, a working memory, and an input/output interface. The CPU executes various types of processing according to processing programs recorded in the program memory. Processes performed by the CPU of the management apparatus 16 are as follows:
· Receiving a medical document from the terminal apparatus 14
· Associating, with each other, the medical document, the hospital name, the patient name, the date, the attributes, and the link information which are received, and transmitting the associated information to the medical-document database 18 to record the associated information in the medical-document database 18
· Receiving a view request from the terminal apparatus 14
· In response to the view request from the terminal apparatus 14, reading medical documents from the medical-document database 18, editing the medical documents, and displaying the edited data on the terminal apparatus 14

The medical-document database 18 records medical documents created in multiple hospitals, in sequence via the management apparatus 16. The medical-document database 18 stores the document name (ID), the hospital name, the patient name, the time (date), the attributes, and link information of a medical document which are associated with each other.

Table 1 illustrates an exemplary data structure stored in the medical-document database 18, in a table format.

**Table 1**

| Document name | Hospital name/ Patient name | Date | Attribute | Link information |
|---|---|---|---|---|
| D001 | A hospital/P | 10/1/2012 | test result | - |
| D002 | C hospital/P | 10/2/2012 | testing | D001 |
| D003 | B hospital/Q | 10/1/2012 | injection | D01 |

For example, Table 1 indicates that a medical document specified with the document name D001 is created in A hospital for a patient P; the creation date of the medical document is 10/1/2012; the attribute of the medical document is the test result; and link information is "not present".

In addition, Table 1 indicates that a medical document specified with the document name D002 is created in C hospital for the patient P; the creation date of the medical document is 10/2/2012; the attribute of the medical document is the testing; and the link information of the medical document describes "D001".

By referring to such a table, the management apparatus 16 may obtain information describing that the medical document D001 is associated with the medical document D002, and gives emphasis indication to the medical document D001 as an associated medical document of the medical document D002. For the patient P, the medical document created in A hospital on 10/1/2012, and the medical document created in C hospital on 10/2/2012 are present. Therefore, a fact that the patient P has been transferred from A hospital to C hospital on 10/2/2012 may be also obtained.

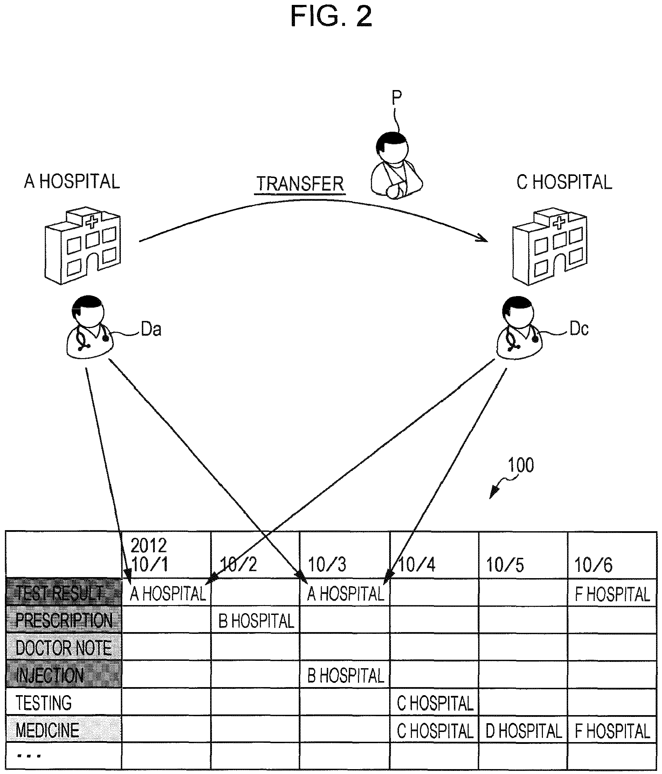

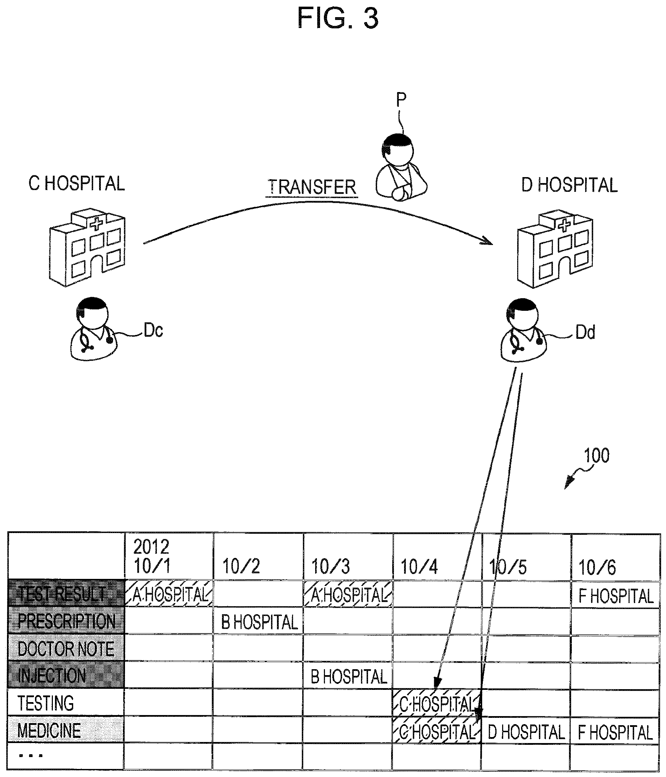

Figs. 2 and 3 illustrate how medical documents are processed in the exemplary embodiment.

In Fig. 2, a patient P takes a medical examination in A hospital, and a doctor Da in A hospital creates various medical documents (including a medical record and a referral form). The medical documents created by the doctor Da in A hospital are converted into electronic formats by using the scanner 12, are supplied to the terminal apparatus 14, and are transmitted to the management apparatus 16 via the network. The management apparatus 16 records the medical documents in the medical-document database 18. In response to a request from the terminal apparatus 14, the management apparatus 16 obtains the creation time (date) and attributes (such as a diagnostic site, treatment information, and a document) of each piece of medical data, and maps the medical data on the same time series (X axis) and attribute items (Y axis) before displaying the medical data. Medical documents created by the doctor Da in A hospital are medical documents whose times are 10/1/2012 and 10/3/2012 and whose attributes are the test result.

Then, the patient P is transferred from A hospital to C hospital, and a doctor Dc in C hospital examines the patient P. The doctor Dc creates medical documents for the patient P. For example, in creation of the medical documents, the doctor Dc refers to the medical documents created in A hospital from which the patient P has been transferred.

At that time, the doctor Dc creates the medical documents by attaching, to the medical documents, links indicating that the medical documents created in A hospital have been referred to in creation of the medical documents. The medical documents created by the doctor Dc are recorded in the medical-document database 18 via the terminal apparatus 14 and the management apparatus 16. In addition, in response to a request from the terminal apparatus 14, the management apparatus 16 obtains the time and attributes (such as a diagnostic site, treatment information, and a document) of each piece of medical data, and displays the medical data on the same time series (X axis) and attribute items (Y axis) as a two-dimensional map 100. The medical documents created by the doctor Dc in C hospital are medical documents whose times are 10/4/2012 and whose attributes are the testing and the medicine.

In Fig. 3, the patient P is further transferred from C hospital to D hospital, and a doctor Dd in D hospital examines the patient P. The doctor Dd creates a medical document for the patient P. In creation of the medical document, the doctor Dd refers to the medical documents created in C hospital from which the patient P has been transferred, and refers to the medical documents created in A hospital which is the previous hospital before the transfer to C hospital.

At that time, the doctor Dd creates the medical document by attaching, to the medical document, links indicating that the medical documents created in C hospital and the medical documents created in A hospital have been referred to in creation of the medical document. The medical document created by the doctor Dd is recorded in the medical-document database 18 via the terminal apparatus 14 and the management apparatus 16. In addition, in response to a request from the terminal apparatus 14, the management apparatus 16 obtains the time and attributes (such as a diagnostic site, treatment information, and a document) of each piece of medical data, and displays the medical data on the same time series (X axis) and attribute items (Y axis) as the two-dimensional map 100. The medical document created by the doctor Dd in D hospital is a medical document whose time is 10/5/2012 and whose attribute is the medicine.

To the medical document created in D hospital, links to the medical documents created in A hospital are attached in creation of the medical document, and links to the medical documents created in C hospital are attached. Assume that the medical document created in D hospital is viewed. The associated medical documents of the medical document, that is, the medical documents viewed by the doctor in creation of the medical document, are associated with the medical document created in D hospital by using the links. Therefore, the link information may be used to easily search for the associated medical documents (in this case, the medical documents created in A hospital and the medical documents created in C hospital). In Fig. 3, the medical documents created in A hospital and the medical documents created in C hospital are highlighted as medical documents associated with the medical document created in D hospital.

### Exemplary Display

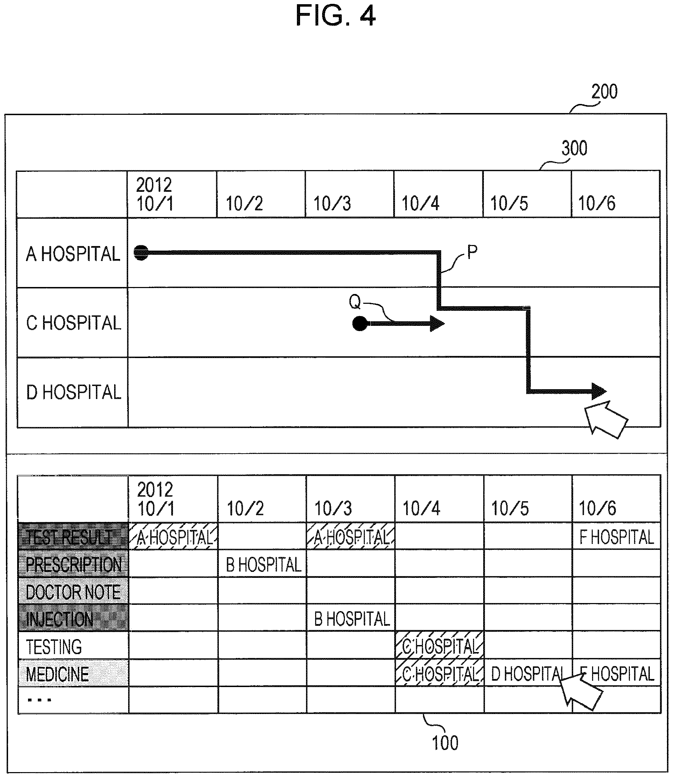

Fig. 4 illustrates an exemplary medical-document view screen 200 displayed on the terminal apparatus 14. In the upper frame of the screen, transfer-between-hospitals history information 300 for patients is displayed, and, in the lower frame of the screen, the two-dimensional map 100 is displayed.

The transfer-between-hospitals history information 300 is displayed in such a manner that the time and hospitals are displayed as a two-dimensional map, and indicates when and in which hospital each patient takes a medical examination. Fig. 4 describes that a patient P takes medical examinations in A hospital from 10/1/2012 to 10/4/2012; the patient P is transferred from A hospital to C hospital on 10/4/2012 and takes a medical examination in C hospital; and the patient P is transferred from C hospital to D hospital on 10/5/2012 and takes a medical examination in D hospital. Fig. 4 also describes that a different patient Q takes a medical examination in C hospital from 10/3/2012. In Fig. 4, a line P indicates the transfer-between-hospitals history of the patient P, and a line Q indicates the transfer-between-hospitals history of the patient Q.

The transfer-between-hospitals history information 300 and the two-dimensional map 100 are associated with each other. When a user selects (for example, touches or clicks on the screen) a 10/5/2012 portion of the line P indicating the transfer-between-hospitals history of the patient P in the transfer-between-hospitals history information 300, the management apparatus 16 automatically selects, in response to the selection, a portion for the medical document created in D hospital for the patient P on 10/5/2012, in a linked manner in the two-dimensional map displayed in the lower frame of the screen. In addition, the management apparatus 16 automatically highlights the medical documents created in A hospital and the medical documents created in C hospital which are associated with the medical document created in D hospital.

Thus, pieces of medical information created in multiple hospitals are displayed as the two-dimensional map 100, and the transfer-between-hospitals history information 300 for patients is displayed on the same screen, enabling visual grasp of a relationship among the medical documents in terms of the hospitals. A document icon indicating that a medical document created in a hospital is present is desirably displayed in such a manner as to be superimposed on the graph in the transfer-between-hospitals history information 300. Selection (a touch or a click) of the document icon may cause information in the medical document to be displayed.

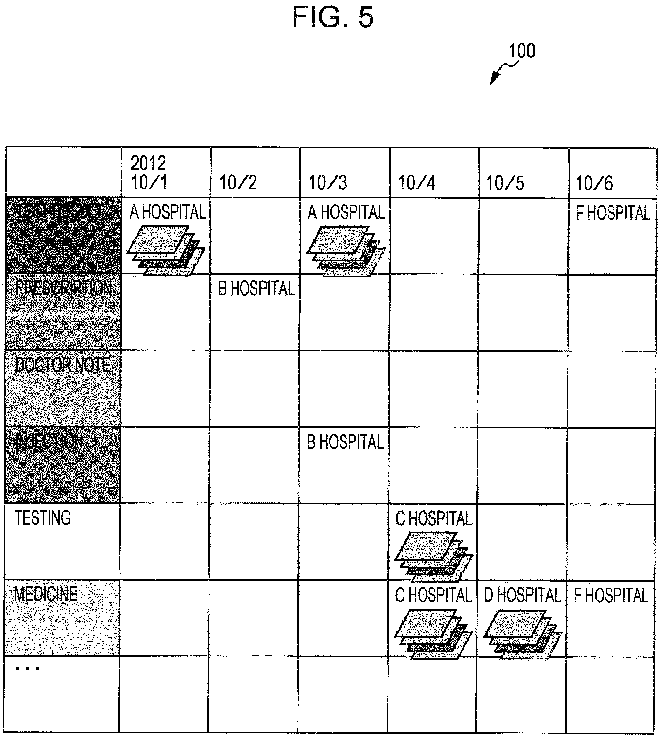

Fig. 5 illustrates an exemplary display of the two-dimensional map 100 according to another exemplary embodiment. When multiple medical documents created by a doctor in a hospital are present, these medical documents are displayed as icons in a hierarchical manner. For example, when multiple medical documents created for the patient P in each of A hospital, C hospital, and D hospital are present, these are displayed as icons in the corresponding cell. In the case where multiple medical documents created by the doctor Dd in D hospital are present, and where a specific medical document among the medical documents is selected, medical documents associated with the medical document are highlighted. Thus, even when multiple medical documents created in each hospital are present, only associated medical documents may be quickly searched for.

The exemplary embodiments are described above. These are not limiting, and various changes may be made.

For example, in the exemplary embodiments, when a doctor views a different medical document, the view operation is recorded so that link information is created, and the created link information is attached to a medical document. Alternatively, a window for selecting an associated document may be activated on the terminal apparatus 14, and an associated medical document may be selected on the window, whereby link information may be created. In short, link information may be created manually on the terminal apparatus 14 or automatically. At that time, the system according to the exemplary embodiments may operate with an electronic medical record system in a linked manner. In creation of an electronic medical record in the electronic medical record system, the management apparatus 16 according to the exemplary embodiments may display the operation screen illustrated in Figs. 4, 5, 6A, and 6B on the terminal apparatus 14 at an appropriate timing. In creation of an electronic medical record, a doctor often refers to other medical documents. Therefore, such a linked system is suitable for actual practices done by a doctor.

In the exemplary embodiments, medical documents for a certain patient are associated with each other. Alternatively, medical documents for different patients may be associated with each other. Specifically, medical documents for patients who are a parent and his/her child may be associated with each other, which will be effective, for example, in searching for an inherited disease.

In the exemplary embodiments, a table as illustrated as Table 1 is used to record medical documents in the medical-document database 18. Other than this, doctor names may be recorded, or a flag which is set to describe whether or not to be published may be recorded. In this case, a medical document not to be published is not allowed to be viewed, and emphasis indication indicating a link target is therefore not given to such a medical document. In addition, when a medical document is to be published, the medical document may be recorded with specification of institutions to which the medical document is to be published. In this case, the medical document is viewed only from the terminal apparatus 14 in a hospital specified as an institution to which the medical document is to be published. Only on the terminal apparatus 14 in such a hospital, emphasis indication is given to the medical document as an associated medical document.

Specifically, in the case where the medical document D001 is created in A hospital, and where A hospital specifies C hospital and D hospital as an institution to which the medical document is to be published, a doctor in C hospital may refer to the medical document D001 to create the medical document D002. When the medical document D002 is referred to by using the terminal apparatus 14 in D hospital, emphasis indication is given to the medical document D001 as an associated document of the medical document D002. In contrast, E hospital is not specified as an institution to which the medical document is to be published. Therefore, even when the medical document D002 is viewed by using the terminal apparatus 14 in E hospital, no emphasis indication is given to the medical document D001 as an associated document of the medical document D002. The management apparatus 16 checks the ID of the terminal apparatus 14 included in a view request from the terminal apparatus 14, against institutions to which the medical document is to be published and which are recorded in the table in an associated manner. The management apparatus 16 determines whether or not emphasis indication is to be given depending on whether or not the ID of the terminal apparatus 14 matches an institution to which the medical document is to be published.

This means that, when a medical document is recorded in the medical-document database 18 with specification of institutions to which the medical document is to be published, information about emphasis indication for associated medical documents displayed on the terminal apparatus 14 dynamically changes depending on whether or not a hospital is specified as an institution to which the medical document is to be published.

Table 2 illustrates exemplary data structure stored in the medical-document database 18 in a table format when institutions to which a medical document is to be published are specified.

**Table 2**

| Document name | Hospital name/ Patient name | Date | Attribute | Link information | Where to be published |
|---|---|---|---|---|---|
| D001 | A hospital/P | 10/1/2012 | test result | - | C hospital |
| D002 | C hospital/P | 10/2/2012 | testing | D001 | all |
| D003 | B hospital/Q | 10/1/2012 | injection | D01 | not to be published |

Table 2 indicates that only C hospital is specified as an institution to which the document D001 is to be published; all of the hospitals are specified as an institution to which the medical document D002 is to be published; and the medical document D003 is specified as a document not to be published. Since the medical document D003 is not to be published, the medical document D003 is not displayed on the two-dimensional map 100 in the first place.

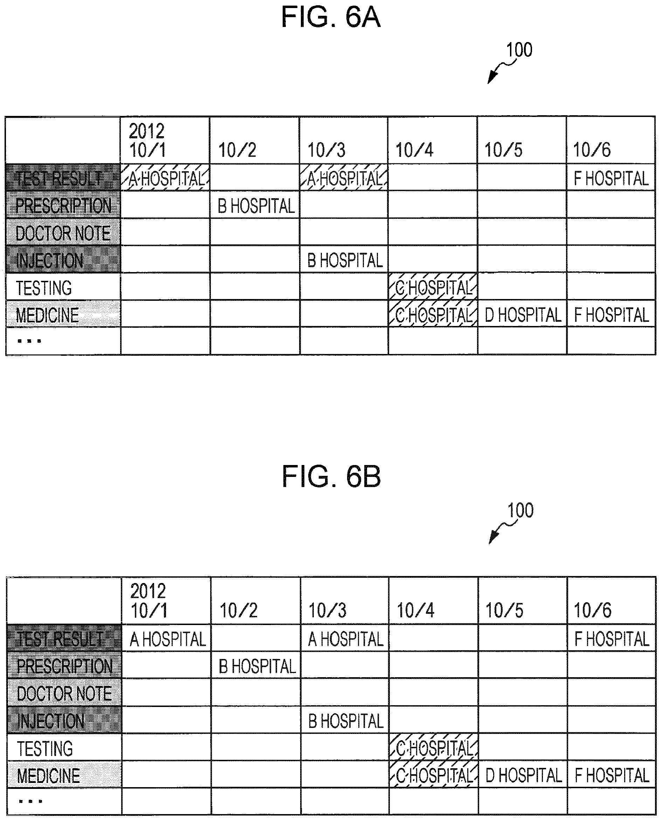

Fig. 6A illustrates an example in which, in the case where both of A hospital and C hospital specify that medical documents are to be published to D hospital, emphasis indication is given when a medical document created in D hospital is viewed. Medical documents created in A hospital and medical documents created in C hospital are present as medical documents associated with the medical document created in D hospital, and are therefore given emphasis indication.

In contrast, Fig. 6B illustrates an example in which, in the case where A hospital does not specify that medical documents are to be published to D hospital and where C hospital specifies that medical documents are to be published to D hospital, emphasis indication is given when the medical document created in D hospital is viewed. Since the medical documents created in A hospital are not published to D hospital, emphasis indication is not given to the medical documents created in A hospital.

The foregoing description of the exemplary embodiments of the present invention has been provided for the purposes of illustration and description. It is not intended to be exhaustive or to limit the invention to the precise forms disclosed. Obviously, many modifications and variations will be apparent to practitioners skilled in the art. The embodiments were chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims and their equivalents.

The description of embodiments may disclose the following matters.
[1] It is an electronic medical record system including: an input unit that receives medical information for a patient; and a display that displays a first medical document and link information, the first medical document and the link information being stored in a database, the link information describing a link to a second medical document associated with the first medical document, the link information being displayed in an emphasized manner.
[2] In the electronic medical record system according to the above [1], the display may display the first medical document and the second medical document on a two-dimensional map with time and attribute, and give emphasis indication to the first medical document and the second medical document on the two-dimensional map.
[3] In the electronic medical record system according to the above [2], the display may further display a transfer-between-hospitals history for each patient on a two-dimensional map with time and hospital, and perform the display operation in such a manner that the two-dimensional map for the medical documents is associated with the two-dimensional map for the transfer-between-hospitals history.
[4] In the electronic medical record system according to any one of the above [1] to [3], the display may further set information about whether or not emphasis indication is to be given, by using information about where the second medical document is to be published.

## Claims

1. A medical-document management apparatus comprising:
a recording unit that records a first medical document and link information in a database, the link information describing a link to a second medical document associated with the first medical document; and
a display that uses the link information to give emphasis indication to the second medical document as a medical document associated with the first medical document.

2. The medical-document management apparatus according to Claim 1,
wherein the display displays the first medical document and the second medical document on a two-dimensional map with time and attribute, and gives emphasis indication to the first medical document and the second medical document on the two-dimensional map.

3. The medical-document management apparatus according to Claim 2,
wherein the display further displays a transfer-between-hospitals history for each patient on a two-dimensional map with time and hospital, and performs the display operation in such a manner that the two-dimensional map for the medical documents is associated with the two-dimensional map for the transfer-between-hospitals history.

4. The medical-document management apparatus according to any one of Claims 1 to 3,
wherein the display further sets information about whether or not emphasis indication is to be given, by using information about where the second medical document is to be published.

5. A medical-document management system comprising:
a medical-document database that stores medical documents for a plurality of hospitals;
a terminal apparatus; and
a medical-document management apparatus according to Claim 2,
wherein the medical-document management apparatus receives a medical document which is converted into an electronic format and which is transmitted from the terminal apparatus, and obtains a date, an attribute, and link information of the medical document so as to record the obtained information in the medical-document database, the link information describing a link to another medical document, and
wherein, in response to a view request from the terminal apparatus, the medical-document management apparatus reads a medical document from the medical-document database so as to transmit the medical document to the terminal apparatus and display the medical document on a two-dimensional map with time and attribute, and gives emphasis indication to associated medical information by using the link information.

6. A program causing a computer to execute a process comprising:
recording a first medical document and link information in a database, the link information describing a link to a second medical document associated with the first medical document; and
using the link information to give emphasis indication to the second medical document as a medical document associated with the first medical document.
